# EUROPEAN PATENT APPLICATION

(11) **EP 4 400 513 A1**
(43) Date of publication of application: **17.07.2024**
(21) Application number: 22797264.3
(22) Date of filing: 01.09.2022
(51) Int. Cl.: C07K 16/28, A61P 37/02, A61K 39/00

(54) **USE OF ANTI-CD6 MONOCLONAL ANTIBODIES IN THE PREVENTION OF CELLULAR AND ORGAN DAMAGE RESULTING FROM HYPER- INFLAMMATORY RESPONSE**

(30) Priority: 08.09.2021 CU 20210074
(71) Applicant: Centro de Inmunologia Molecular, Playa, La Habana 11300 (CU)
(72) Inventor: GARCÍA VEGA, Yanelda de los Ángeles, La Habana 11300 (CU); ABDO CUZA, Anselmo Antonio, La Habana 11300 (CU); CORREA PADILLA, Jorge Miguel, La Habana 10200 (CU); ÁLVAREZ BENITO, Octavio, La Habana 10200 (CU); LEÓN MONZÓN, Kalet, La Habana 17100 (CU); SUÁREZ LÓPEZ, Juliette María, La Habana 10400 (CU)
(74) Representative: Michalski Hüttermann & Partner Patentanwälte mbB
(86) International application number: PCT/CU2022/050010
(87) International publication number: WO 2023/036349

(57) **Abstract**

The present invention relates to the fields of biotechnology and medicine. In particular it describes the use of anti-CD6 monoclonal antibodies that specifically bind to CD6 domain 1 in the prevention of the hyper-inflammatory response that is triggered by medical procedures resulting from the passage of blood through an extracorporeal circuit. The anti-CD6 antibodies of the present invention show a reduction in the degree of hyper-inflammation through a lower concentration of IL-6, CRP and LDH; with the consequent prevention of cellular and organ damage.

## Description

### FIELD OF TECHNOLOGY

The present invention relates to the branches of biotechnology and medicine; in particular, to the use of anti-CD6 monoclonal antibodies (mAbs) in the prevention of cellular and organ damage resulting from a hyper-inflammatory response that is triggered by, or affects the final outcome of, specific medical procedures.

### BACKGROUD

The use of extracorporeal organ support was one of the most important advances in medicine in the last century. The most frequently used are extracorporeal circulation (ECC) and renal replacement therapies; both methods involve the circulation of blood outside the body through artificial circuits.

ECC or cardiopulmonary bypass or heart-lung machine/pump, as it is also known, and extracorporeal membrane oxygenation (ECMO) is applied in different circumstances, such as cardiovascular surgery and support in patients with severe cardiorespiratory dysfunction. Renal replacement therapies (dialysis, haemofiltration, haemodiafiltration and others) are widely used in acute and chronic renal failure (Durmaz I et al. (1999), Thorac Cardiovasc Surg. 118:306-315).

Different complications have been described during and after these prpcedures, including cardiac surgery which itself provokes a vigorous inflammatory response, which has important clinical implications. Since the early days of cardiac surgery using CPB, complications such as fever, pericarditis and pleuritis have been described (Ribot S et al. (1971) J Thorac Cardiovasc Surg. 62:59-62).

In addition, chronic inflammation promotes important adverse consequences. Indeed, persistent low-grade inflammation is recognised as a major contributor to the risk of mortality, cardiovascular events (including early atherosclerosis), kidney diseaseprogression, osteoporosis and other adverse outcomes; both in chronic kidney disease and chronic dialysis. (Nowak K and Chonchol M (2018) Semin Dial. 31 (4):388-397; (Cobo G et al. (2018) Nephrol Dial Transplant 33:iii35-iii40). In renal replacement procedures, can occur both medium- and long-term complications.

After decades of research, post-cardiac surgery inflammatory response syndrome (PCIRS) was described in 1981. In cardiac surgery patients with the use of CPB, PCIRS occurs with higher frequency and severity. A group of factors such as anaesthetic techniques, perfusion techniques, exposure or contact of blood with foreign surfaces (CPB circuit), surgical technique and post-operative care activate, through mediators, an inflammatory response. This response, in a group of patients, can be counterproductive. The organ dysfunction caused by the inflammatory response syndrome, which includes myocardial, pulmonary, renal and hepatic dysfunction among others, can progress to multiple organ dysfunction syndrome in 2 - 11% of operated patients, with mortality between 41-78 % (Uyar IS et al. (2013), Cardiovasc J Afr. 24: 322-326). Although to a lesser extent, severe neurological injuries after cardiac surgery are also described in 6.1% (3.1% focal and 3% diffuse injuries), including central nervous system dysfunction (Suárez Gonzalo L et al. (2002 Med Intensiva 26(6):292-303).

The contact of blood with non-endothelial surfaces of the CPB circuit is responsible for the onset of the inflammatory response syndrome, involving cytokines, coagulation factors, fibrinolysis, complement and the cellular immune system. Different authors have demonstrated elevated levels of interleukins such as IL-1, IL-6, IL-8 and TNF-α in post-operative cardiac surgery using CPB, which have been associated with myocardial dysfunction, haemodynamic instability, fever and lung injury (Uyar IS et al. (2013), Cardiovasc J Afr. 24: 322-326□ Durmaz I et al. (1999), Thorac Cardiovasc Surg. 118:306-315). Also, the use of haemodialysis in patients with chronic kidney disease may be accompanied by elevated levels of inflammatory biomarkers such as IL-6 and C-reactive protein (CRP) compatible with low-grade inflammatory process (Nowak K and Chonchol M (2018) Semin Dial. 31 (4):388-397). In summary, the use of extracorporeal organ support can be accompanied by acute or persistent inflammatory or hyper-inflammatory responses, depending on the frequency of extracorporeal organ support use, which can induce organ dysfunction with adverse outcomes.

In the face of any aggression, the body launches a response with an initial inflammatory phase, mediated by humoral and cellular factors, characterised by a restricted and localised response that tends to limit tissue damage; it isolates, destroys and eliminates the infectious microorganisms and activates the repair processes necessary for the return of the damaged organ to its normal function. Similar to the ECC circuit, in the immune response of injured patients, monocytes and endothelial cells initially release a number of pro-inflammatory cytokines into the injured area. TNFα and IL-1 are the first cytokines secreted after trauma, they stimulate several immunocompetent cells and induce the release of pro-inflammatory cytokines (IL-6, IL- 8 and interferon gamma). In complex injured patients with bone and soft tissue injuries diagnosed with systemic inflammatory response syndrome (SIRS), there are IL-6 elevations of up to 200-300 pg/mL (Tanaka T et al. (2016) Immunotherapy 8(8):959- 970).

Advances in anaesthetic-surgical techniques and the use of more biocompatible circuits have shown some benefit, but have not eliminated PCIRS. Thoracic epidural anaesthesia combined with general anaesthesia for aorto-coronary bridges decreases the peri-operative stress response, which may decrease post-operative pulmonary and myocardial damage. However, thoracic epidural anaesthesia does not significantly decrease the cytokine response during CPB (Molina Méndez FJ. (2004) Arch Cardiol Mex.74 (S-2):505-508).

A number of drug strategies exist to impact on different points of the inflammatory reaction, such as the use of steroids, serine protease and phosphodiesterase inhibitors, antioxidants (allopurinol, N-acetylcysteine, alpha lipoic acid) and anti-C5a-MAA. These drugs have been used before, during or after the use of CPB. Technological actions such as the use of haemofiltration/hemoperfusion and anti-cytokine filters have also been used. Although these procedures and drugs have achieved favourable responses, they have not demonstrated the expected effectiveness and still continue to report a high frequency of morbidity and mortality associated with the use of CPB (Ribot S et al. (1971) J Thorac Cardiovasc Surg. 62:59-62 Uyar IS et al. (2013), Cardiovasc J Afr. 24: 322-326).

The use of alpha lipoic acid administered in the main CPB solution during the surgical procedure decreases IL-6 and CRP concentrations, but has not demonstrated clinical impact on reducing complications or mortality (Uyar IS et al. (2013), Cardiovasc J Afr. 24: 322-326). Neither does the use of steroids peri- or intraoperatively in cardiac surgery reduce major complications or 30-day mortality. In addition, it includes important adverse events such as the risk of infections and myocardial damage (Pasquali SK et al. (2012) Pediatrics. 129(2):e385-e391 Whitlock RP et al. (2015) Lancet 386(10000):1243-1253 Graham EM et al. (2019) J Am Coll Cardiol. 74(5):659- 668 Lomivorotov V et al. (2020) JAMA. 323(24):2485-2492).

Urinary trypsin inhibitor administered intravenously trans- and post-operatively decreases concentrations of TNF-α, IL-1β, IL-6, IL-8 and transaminases with a consequent decrease in the duration of ventilation and hospitalisation time in the Intensive Care Unit (ICU), but it is not known whether the decrease in these parameters decreases the morbi-mortality of treated patients (Xu H, et al. (2017) Euro Rev Medical and Pharmacol Sc.; 21: 2220-2225; G. He, et al. (2018) Herz https://doi.org/10.1007/s00059-018-4732-0). Pexelizumab (anti-C5 mAb) also showed no clinically significant effect in reducing morbidity and mortality and was not free of adverse reactions including infections with sepsis among them (Shernan SK et al. (2004) Ann Thorac Surg. 77:942-50).

The use of anti-factor D in monkeys undergoing cardiopulmonary bypass demonstrated inhibition of complement activation, CD11b expression on neutrophils and monocytes as well as decreased increases in plasma IL-6 concentration, compared to the control group that did not receive the MCA as well as markers of myocardial damage and renal function and creatinine. However, no human study was found in the literature reviewed in which the use of anti-factor D demonstrated the effect on cytokine reduction, as prevention and modulation of the hyper-inflammatory response produced by the flow of blood through the extracorporeal circuit and its consequent cellular damage.

Patent PCT/IB2021/052793 describes the use of nondepleting anti-CD6 mAbs that specifically bind CD6 domain I in the treatment of cytokine storm caused by infectious agents such as viruses and bacteria. Patients with pneumonia treated with these mAbs showed reduced levels of IL-6, IL-17, TNFα and CRP, leading to improved lung function parameters, preventing progression to critical or severe stages, and improved ICU management.

So far, there is no previous information on the use of mAb against CD6 in the prevention of cellular and organ damage resulting from non-infectious hyper-inflammatory responses triggered by medical procedures resulting from the flow of blood through an extracorporeal circuit or trauma.

Unexpectedly, the inventors of the present invention, by employing the mAbs described here in patients in need of CPB or who suffered trauma, achieved a reduction in the degree of hyper-inflammation through a lower concentration of IL-6, CRP, and markers of organ function and damage such as LDH and GPT, with consequent prevention of cellular and organ damage, with clinical impact by reducing overall morbidity, multiple organ failure and post-operative mortality.

### BRIEF DESCRIPTION OF THE INVENTION

In one embodiment the present invention relates to the use of mAbs that specifically recognise CD6, in the prevention of cellular and organ damage resulting from a hyper- inflammatory response that is triggered by highly invasive medical procedures, or affects the final outcome of such procedures. In particular, these mAbs are non- depleting and bind specifically to CD6 domain 1. These mAbs comprise SEQ ID NO. 1 and 2 sequences or have 98% identity to these and in this case comprise SEQ ID NO.1 and SEQ ID NO. 3 sequences. Alternatively, these mAbs may comprise SEQ ID NO.4 and 5 sequences or have 98% identity to them and then comprise SEQ ID NO. 4 and SEQ ID NO. 5. 4 and SEQ ID NO. 6. In particular, these anti-CD6 mAbs are humanised IgG1 isotype, preferably, the itolizumab mAb.

In particular, the present invention relates to the use of the mAbs described herein to prevent cellular and organ damage arising from the following medical procedures or conditions: cardiac surgery, lung transplant surgery, giant brain aneurysm surgery, vascular surgery, extracorporeal membrane oxygenation, mechanical circulatory support, haemofiltration, renal dialysis and haemodiafiltration, damage control surgery, trauma, polytrauma, burns, necrotising peritonitis, acute inflammatory response syndrome, without being limited to these.

In a further aspect, the present invention provides for the combination of the anti-CD6 mAbs with agents that are selected from the group comprising anaesthetics, sedatives, relaxants, antivirals, antibiotics, erythropoietin, agents that block the interaction between IL-6 and the IL-6 receptor and agents that block the interaction between IL1 and the IL1 receptor. Such administration can be done serially or concomitantly.

In a further embodiment, the present invention relates to a method of treating a subject undergoing CPB comprising administering intravenously or subcutaneously mAbs comprising the amino acid sequences shown in SEQ ID NO. 1 and 2 or SEQ ID NO. 4 and 5 or mAbs with an amino acid sequence having 98% identity thereto and comprising SEQ ID NO. 1 and SEQ ID NO. 5. 4 and 5 or the mAbs with an amino acid sequence having 98% identity to them and comprising SEQ ID NO. 1 and SEQ ID NO.3 sequences or the sequences SEQ ID NO. 4 and SEQ ID NO. 6; in a dose range between 0.3 mg/kg body weight to 6 mg/kg body weight. These MDAs are administered to the subject at least once, the time between two consecutive administrations is between three and seven days.

### DETAILED DESCRIPTION OF THE INVENTION

### Pharmaceutical compositions

The mAb used in the present invention are administered as part of pharmaceutical compositions containing the mAb as the active substance and as an appropriate excipient buffered physiological solution, similar to others used to formulate mAb for intravenous or subcutaneous use. In particular, the itolizumab sequences are described in patent US 6,572,857 B1 and 8,524,233 and are shown in detail in Table 1.

**Table 1. Protein sequence of itolizumab.**

| SEQ ID NO. | DESCRIPTION | SEQUENCE |
|---|---|---|
| 1 | Variable region of Heavy Chain | EVQLVESGGGLVKPGGSLKLSCAASGFKFSRYAMSWVRQAPGKR LEWVATISSGGSYIYYPDSVKGRFTISRDNVKNTLYLQMSSLRSEDT AMYYCARRDYDLDYFDSWGQGTLVTVSS |
| 2 | Variable region of Light Chain | DIQMTQSPSSLSASVGDRVTITCKASRDIRSYLTWYQQKPGKAPKT LIYYATSLADGVPSRFSGSGSGQDYSLTISSLESDDTATYYCLQHGE SPFTLGSGTKLEIK |
| 3 | Variable region of Light Chain | DIQMTQSPSSLSASVGDRVTITCKASRDIRSYLTWYQQKPGKAPKT LIYYATSLADGVPSRFSGSGSGQDYSLTISSLESDDTATYYCLQHGE SPFTFGSGTKLEIKRA |
| 4 | Heavy Chain | EVQLVESGGGLVKPGGSLKLSCAASGFKFSRYAMSWVRQAPGKR LEWVATISSGGSYIYYPDSVKGRFTISRDNVKNTLYLQMSSLRSEDT AMYYCARRDYDLDYFDSWGQGTLVTVSSASTKGPSVFPLAPSSKS TSGGTAALGCLVKDYFPEPVTVSWNSGALTSGVHTFPAVLQSSGL YSLSSVVTVPSSSLGTQTYICNVNHKPSNTKVDKKVEPKSCDKTHT CPPCPAPELLGGPSVFLFPPKPKDTLMISRTPEVTCVVVDVSHEDP EVKFNWYVDGVEVHNAKTKPREEQYNSTYRVVSVLTVLHQDWLN GKEYKCKVSNKALPAPIEKTISKAKGQPREPQVYTLPPSRDELTKNQ VSLTCLVKGFYPSDIAVEWESNGQPENNYKTTPPVLDSDGSFFLYS KLTVDKSRWQQGNVFSCSVMHEALHNHYTQKSLSLSPGK |
| 5 | Light Chain | DIQMTQSPSSLSASVGDRVTITCKASRDIRSYLTWYQQKPGKAPKT LIYYATSLADGVPSRFSGSGSGQDYSLTISSLESDDTATYYCLQHGE SPFTLGSGTKLEIKRTVAAPSVFIFPPSDEQLKSGTASVVCLLNNFYP REAKVQWKVDNALQSGNSQESVTEQDSKDSTYSLSSTLTLSKADY EKHKVYACEVTHQGLSSPVTKS |
| 6 | Light Chain | DIQMTQSPSSLSASVGDRVTITCKASRDIRSYLTWYQQKPGKAPKT LIYYATSLADGVPSRFSGSGSGQDYSLTISSLESDDTATYYCLQHGE SPFTFGSGTKLEIKRARTVAAPSVFIFPPSDEQLKSGTASVVCLLNN FYPREAKVQWKVDNALQSGNSQESVTEQDSKDSTYSLSSTLTLSK ADYEKHKVYACEVTHQGLSSPVTKSFNRGEC |

### Therapeutic application and treatment methods

The present invention relates to the use of anti-CD6 mAbs in the prevention of a hyper- inflammatory response that is triggered by, or affects the outcome of, specific medical procedures, including cardiac surgery, lung transplant surgery, giant brain aneurysm surgery and vascular surgery, extracorporeal membrane oxygenation (ECMO), mechanical circulatory support (MCS), renal replacement therapies such as haemofiltration, dialysis on therapy or iteration, haemodiafiltration, damage control surgery, trauma, polytrauma, burns, necrotising peritonitis, acute inflammatory response syndrome.

In particular, it relates to the use under the above conditions of the anti-CD6 mAbs whose amino acid sequence is shown in SEQ ID NO. 1 (heavy chain variable region) and SEQ ID NO. 2 (light chain variable region) or with sequences having 98% identity thereto and comprising SEQ ID NO. 1 (heavy chain variable region) and SEQ ID NO. 3 (light chain variable region) according to the present invention. In addition, with anti-CD6 mAb having heavy and light chain regions comprising the amino acid sequences set in SEQ ID NO.4 (heavy chain) and SEQ ID NO.5 (light chain) or sequences with 98% identity thereto according to the present invention. An anti-CD6 mAb comprising or consisting of SEQ ID NO: 4 and 6 is included in the present invention.

The term nondepleting anti-CD6 Ac, used in the present invention, means an AcM that recognizes CD6 and that once it binds to CD6, does not induce Ac-dependent cellular cytotoxicity (ADCC), complement-dependent cellular cytotoxicity (CDC) or otherwise promote lysis or death of CD6-expressing cells. Therefore, a particular advantage of the proposed treatment is the non-induction of immunodeficiency in the patient, unlike other therapies based on steroids, mAb or other immunosuppressants. The preservation of some immuno-competence in the treated patient reduces the possibility of emergence of other opportunistic infections, which are very common in intensive therapies.

Patients receiving anti-CD6 mAb therapy may be admitted to an intensive care unit, but may also be in a conventional ward or outpatient clinic. Adult, paediatric and pregnant patients are eligible for anti-CD6 mAb therapy.

The antibody shall be administered to subjects (such subjects are vertebrates, such as humans), intravenously or subcutaneously, in a dose range between 0.3 mg/kg body weight and 6 mg/kg body weight, corresponding to 25 and 420 mg total respectively. The antibody should be administered at least once to the patient and the time between two consecutive administrations should be between three and seven days. The dose and schedule to be used for different anti-CD6 mAbs may be adjusted by assessing the IL-6 concentration or the ability to inhibit in vitro or in vivo CD6+ T and/or B cell activation, or without inducing their elimination/depletion as in the specific case of itolizumab mAb.

Serial or concomitant combination of anti-CD6 mAbs with anaesthetics, sedatives or relaxants as well as antiviral or antibiotic therapies as appropriate to the infection resulting from hyper-inflammation as a consequence of CPB or trauma is also the subject of the present invention.

For patients on ECMO, mechanical circulatory support and haemofiltration, sedation with hypnotic agents and, if necessary, muscle relaxation with non-depolarising relaxants will be used.

Anti-CD6 mAbs can be combined with any anaesthetic, sedative, relaxant or antibiotic permitted for use in humans depending on the patient's medical condition. Possible antivirals to combine include, but are not limited to, ribavirin, type 1 and type 2 interferons, as well as protease inhibitors (including but not limited to boceprevir, telaprevir, simeprevir), reverse transcriptase inhibitors (including but not limited to lazidovidin, abacabir, lamivudine, emtricitabine, tenofovir, nevirapine, efavirenz, etravirenz, etravir), nevirapine, efavirenz, etravir, nevirapine, efavirenz, etravirine), polymerase inhibitors (including but not limited to sofosbuvir, ledipasvir, velpatasvir, saquinavir, ritonavir, indinavir, nelfinavir, lopinavir, nelfinavir, lopinavir, atazanovir, fosamprenavir, tipranavir, darunavir) and NS5A protein (including but not limited to elbasvir/grazoprevir) or combinations thereof. The antibiotic or antiviral regimens to be used will be those conventionally used for each drug, while the anti-CD6 mAb regimen will be as described above.

Also subject to the present invention is the serial or concomitant combination of an anti-CD6 Mab with therapeutic agents that block the interaction between IL-6 and IL-6R or between IL-1 and IL-1R. In particular, the treatments of the additional therapies should be short enough to contribute to the reduction of hyper-inflammation, but without inducing an immunosuppression that facilitates the spread of bacteria and viruses in the patient.

In another aspect, the serial or concomitant combination of an anti-CD6 mAb with erythropoietin is the subject of the present invention. In particular, in the protection of kidney damage primarily in patients on dialysis.

### BRIEF DESCRIPTION OF THE FIGURES

**Figure 1****.** Inflammatory profile (LDH, IL-6 and CRP concentrations) of a patient treated with itolizumab at a dose of 1.6 mg/kg body weight.
**Figure 2****.** Comparison of IL-6 concentrations in patients undergoing surgery with CPB without and with itolizumab treatment at a dose of 1.6 mg/kg body weight.
**Figure 3****.** CRP concentrations in the different conditions evaluated compared to treatment with itolizumab at a dose of 1.6 mg/kg body weight. Normal level < 6 pg/L
**Figure 4****.** LDH concentrations in patients undergoing surgery with CPB not treated with itolizumab compared to treatment with itolizumab at a dose of 1.6 mg/kg body weight.Reference range: 240-480 IU/L
**Figure 5****.** Liver profile (ALT, FAL and total bilirubin concentrations) of a patient treated with itolizumab at a dose of 1.6 mg/kg body weight. Normal levels ALT < 40 IU/L, ALF 39-117 IU/L, total bilirubin < 20µg/L.
**Figure 6****.** Renal profile (creatinine and urea concentrations) of a patient treated with itolizumab at a dose of 1.6 mg/kg body weight. Normal creatinine levels in men: 62- 106 µmol/L and women: 44-80 µmol/L, urea: 1.7-8.3 µmol/L.
**Figure 7****.** IL-6 and LDH concentrations in a patient treated with itolizumab at a dose of 0.3 mg/kg body weight.
**Figure 8****.** Comparison of IL-6 concentrations in the different conditions tested compared to itolizumab treatment at 0.3 mg/kg body weight.
**Figure 9****.** IL-6 and lactate concentration in a post-trauma patient undergoing orthopaedic surgery treated with itolizumab at a dose of 1.6 mg/kg body weight. Normal lactate levels 0.5-1.6 mmol/L.

### EXAMPLES

### Example 1. Demonstration of the reduction of the degree of hyper-inflammation in a patient with mitral valve replacement surgery and CPB treated with itolizumab (1.6 mg/kg body weight).

A patient undergoing mitral valve replacement surgery was administered 1.6mg/kg body weight of itolizumab AcM (100 mg) in 250 mL of saline and 2 mg of midazolam as anaesthetic pre-medication. After basic monitoring, a cannula was placed in the radial artery for invasive blood pressure monitoring. After monitoring heart rate, invasive blood pressure, electrocardiogram and oxygen saturation, anaesthetic induction was performed with lidocaine 70 mg, fentanyl 350 µg, midazolam 8 mg, followed by atracurium 35 mg. Laryngoscopy and easy intubation with 8.0 endotracheal tube, coupled to anaesthesia machine on volume-controlled ventilation. A central venous line was placed by percutaneous puncture of the internal jugular vein. Maintenance was performed with balanced anaesthesia with isofluorane and a continuous infusion of fentanyl at 5µg/kg/h, midazolam 0.15 mg/kg/h and atracurium 0.2 mg/kg on demand. A continuous 4 mg magnesium solution was administered over one hour.

Sternal opening without complications. The ascending aorta and superior and inferior vena cava were cannulated. After heparinisation 5mg/kg, CPB was started and a mean flow between 1.8 and 2.4 L/m2 of body surface area was maintained, with a mean arterial pressure between 60 and 70 mmHg. Aorta was clamped and hyperkalemic cardioplegia was used for myocardial protection. Prosthetic valve replacement was performed without complications. Aortic clamping time of 64 minutes. Patient active in sinus rhythm. Normal internal environment, vital signs within physiological parameters, exits bypass without haemodynamic support. CPB time 156 minutes. Heparin is reversed.

Surgery was successfully completed. The patient was transferred to intensive care intubated, haemodynamically stable. Heart rate 76 beats per minute, blood pressure 110/60 mmHg arterial oxygen partial pressure 99%. The patient progressed stably post-operatively with no signs or symptoms of organ damage. There were no subsequent infections and he was discharged seven days post-operatively.

To assess IL-6, LDH and CRP, ALT, FAL, total bilirubin, creatinine and urea concentrations, samples were taken prior to surgery or itolizumab treatment (0 hours), as well as at 12, 24, 48 and 168 hours.

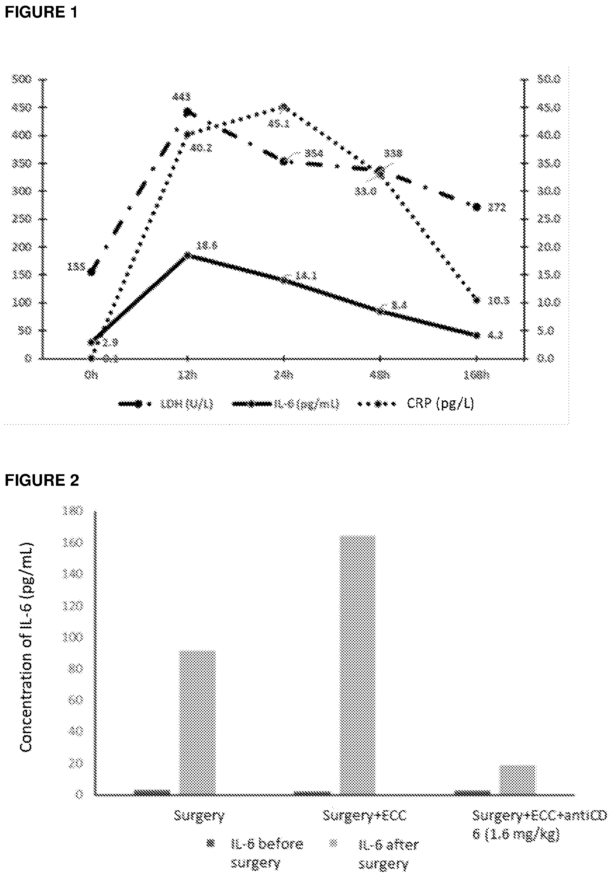

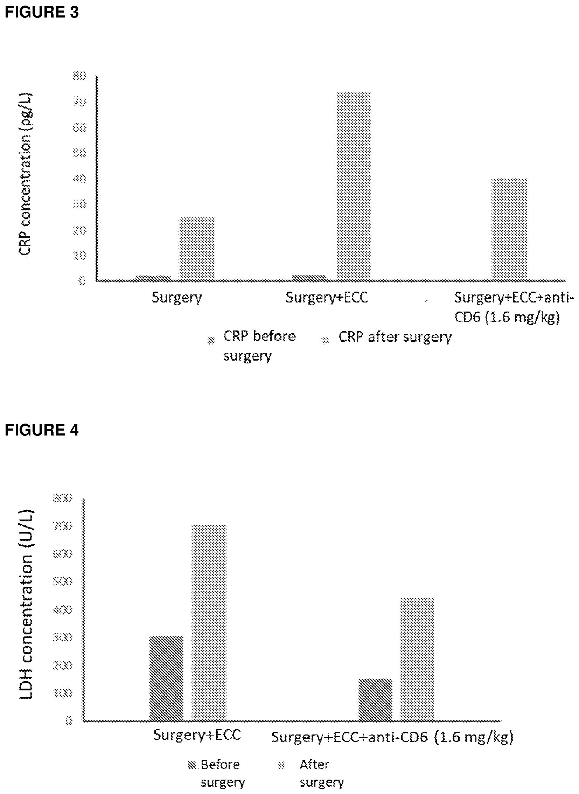

In the inflammatory profile, after surgery with CPB (Figure 1) an IL-6 concentration of 18.6 pg/mL was observed at 12 hours, which is above the reference levels (≤ 7 pg/mL); but much lower (9 times less) than the mean found in the group of patients undergoing surgery with CPB who did not receive itolizumab (Figure 2), so that treatment with itolizumab (1.6 mg/kg body weight) manages to control the increase in IL-6 concentration produced by CPB, even below that of surgery alone. Also, the CRP concentration increased reaching a maximum level at 24 hours (45.1 pg/L, Figure 1); but when comparing the results with those of the group of patients who required CPB (Figure 3), it was found that treatment with itolizumab at a dose of 1.6 mg/kg body weight, the CRP concentration increased less (1.8 times lower).

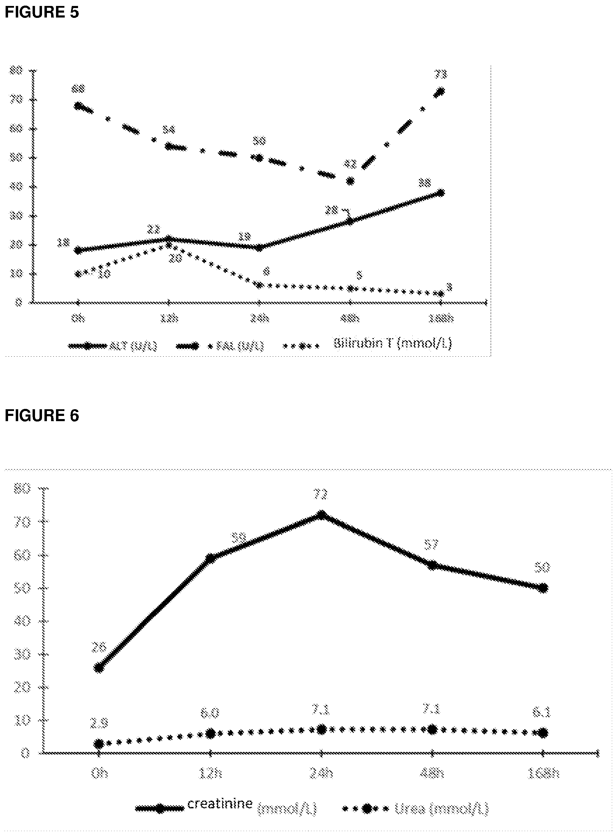

Additionally, treatment with itolizumab (1.6 mg/kg body weight) prevented the increase of LDH concentrations above the upper reference range as a reflection of no cellular or organ damage, contrary to what happens in patients undergoing surgery with CPB (Figure 4). Regarding the liver profile (Figure 5), the concentrations of the measured parameters (ALT, FAL, total bilirubin) always remained within the normal reference range, indicating that there was no liver damage. Creatinine and urea concentrations remained within the normal range, indicating that no renal damage was detected (Figure 6).

### Example 2. Demonstration of the reduction of the degree of hyper-inflammation in a patient with mitral valve replacement surgery treated with itolizumab (0.3 mg/kg body weight).

A patient undergoing mitral valve replacement surgery was administered 0.3 mg/kg body weight of itolizumab AcM (25 mg) in 50 mL of saline and 2 mg of midazolam as anaesthetic pre-medication. After basic monitoring, a cannula was placed in the radial artery for invasive blood pressure monitoring. In the operating room, after monitoring heart rate, invasive blood pressure, electrocardiogram and oxygen saturation, anaesthetic induction was performed with lidocaine 80 mg, fentanyl 400 µg, midazolam 8 mg, followed by atracurium 40 mg. Laryngoscopy and easy intubation with endotracheal tube 8.5, coupled to anaesthesia machine on volume-controlled ventilation. A central venous line was placed by percutaneous puncture of the internal jugular vein. Maintenance was performed with balanced anaesthesia with isofluorane and a continuous infusion of fentanyl at 5 µg/kg/h, midazolam 0.15 mg/kg/h and atracurium 0.2 mg/kg on demand. A continuous 4mg magnesium solution was administered over one hour.

Sternal opening without complications. The ascending aorta and superior and inferior vena cava were cannulated. After heparinisation 5mg/kg, CPB was started and a mean flow between 1.8 and 2.4 L/m2 of body surface area was maintained, with a mean arterial pressure between 60 and 70 mmHg. Aorta was clamped and hyperkalemic cardioplegia was used for myocardial protection. Prosthetic valve replacement was performed without complications. Aortic clamping time was 71 minutes. Patient active in sinus rhythm. Normal internal environment, vital signs within physiological parameters, exits bypass without haemodynamic support. CPB time 95 minutes. Heparin is reversed.

Surgery was successfully completed. The patient was transferred to intensive care intubated, haemodynamically stable. Heart rate 80 beats per minute, blood pressure 120/70 mmHg, arterial oxygen partial pressure 100%.

To determine IL-6 and LDH concentration, samples were taken prior to surgery or itolizumab treatment (0 hours), 12 and 24 hours.

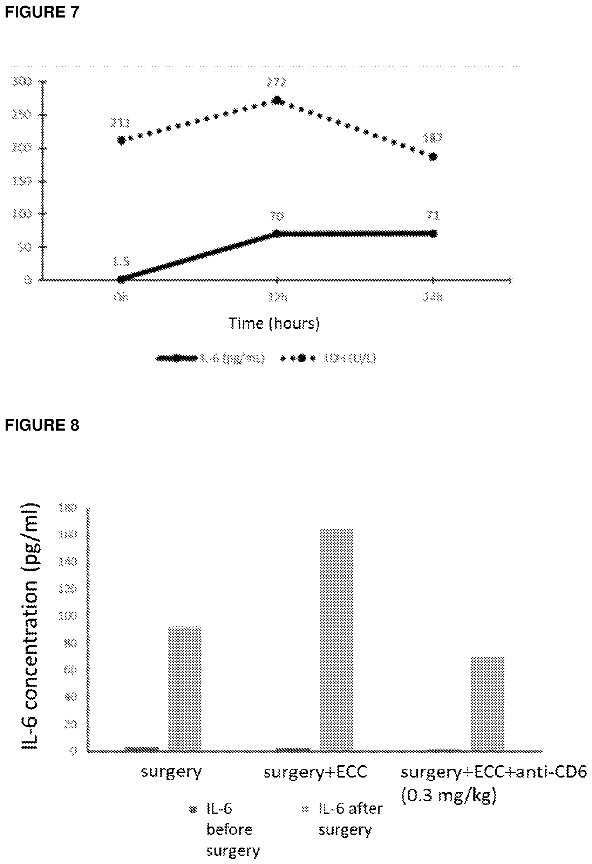

As shown in figure 7, after surgery with CPB, IL-6 concentration increased at 12 hours, while LDH concentration was within the normal reference range. Comparing the results of the effect of itolizumab (0.3 mg/kg body weight) on IL-6 concentration to the controls tested (figure 8) also confirmed a significant attenuation of the increase in this cytokine that results from the surgical procedure alone or that exacerbates the flowof blood through CPB. No permanent complications or subsequent infections were noted. Ten days post-operatively the patient was discharged from the hospital. Treatment with itolizumab attenuated the inflammatory response as evidenced by monitoring of IL-6 and CRP concentration; evidence of prevention of cellular and organ damage.

### Example 3. Demonstration of the reduction in the degree of hyper-inflammation prior to surgery in a patient who suffered a road traffic accident.

A patient who had suffered a traffic accident was admitted to hospital approximately 1 hour later, conscious with great psychomotor agitation, clear and coherent language with peri-traumatic amnesia, with stigmata of frontal cranial trauma, and multiple friction burns on the upper limbs and left shoulder region. Respiratory system: preserved thoracic expansibility, no stigmata of trauma, preserved vesicular murmur, no rales, respiratory rate: 23/min. Cardiovascular system: pale, sweaty patient with generalised skin coldness. Tachycardic rhythmic sounds with good tone, heart rate 102/min. Abdomen soft, depressible, non-painful, no peritoneal reaction. In the left lower limb there was an increase in volume with deformity and angulation at the level of the middle third of the thigh and leg with marked external rotation and flexion, apparent shortening of the limb. Palpation revealed abnormal mobility in the middle third of the tibia and femur. Thread-like pulses present, coldness and sweating in the extremities. Sensitivity preserved and active and passive motility preserved. When assessing his physiological status, he was considered to be in a borden-line stage of complex injured patients and based on the Consensus Conference between the American College of Chest Physicians and the Society of CriticalCare Medicine, which defines the diagnosis of SIRS as being characterised by the presence of two or more of the following signs: Temperature < 36 °C or> 38 °C; Heart rate > 90 beats/min; Respiratory rate > 20 resp/min or pCo2< 32 mmHg and Leukocytes > 12.000/mm3 or< 4,000/mm3 or> 10% immature forms. It was decided to administer mAb itolizumab (100 mg) at a rate of 1.6 mg/kg b.w. diluted in 250 mL of physiological saline solution, in continuous infusion with infusion pump for two hours in the trans-operative period. A retrograde non-rhinged steel endomedullary nailing of the femur fracture and non-rhinged steel endomedullary nailing of the tibia fracture were performed by means of a single infra patellar approach with a surgical time of 1h 27min.

Serum samples were taken for determination of IL-6 concentration at 1, 3, 6, 12 and 24 hours after the accident.

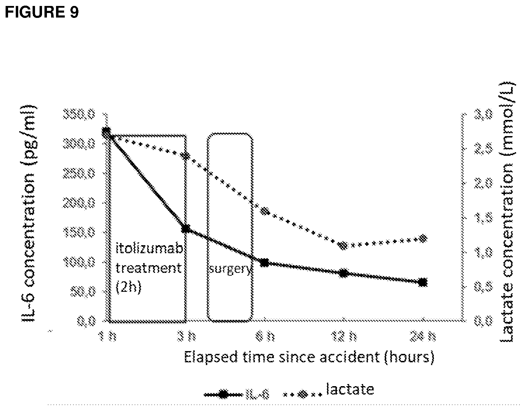

The 50% decrease in IL-6 concentration after 2 hours of itolizumab infusion, together with the physiological stabilization of the patient (decrease in lactate concentration) allowed definitive surgery to be performed in the first hours after trauma. In the first 24 hours, a decrease of up to 80% in IL-6 concentration was observed with improvement of humoral parameters, especially lactate (Figure 9). This, together with the evident clinical improvement of the patient, meant that the patient did not develop SIRS, with the consequent prevention of evolution towards organ dysfunction or death. This finding also allows definitive fixation to be performed between 48 and 72 hours after the trauma, which reduces the waiting time, which conventionally ranges between 5 and 10 days, with a corresponding reduction in hospital stay.

## Claims

1. Use of a monoclonal antibody (MAb) that specifically recognize to CD6 for preventing the cellular and organic damage derived from a hyper-inflammatory response triggered by highly invasive medical procedures, or affects the final result of such procedures.

2. The use according to claim 1, wherein the MAb is a non-depleting MAb.

3. The use according to claim 1, wherein the MAb that specifically binds to domain 1 of CD6 comprises the sequences SEQ ID NO 1 and 2 or having 98% identity to SEQ ID Nos: 1 and 2.

4. The use according to claim 3, wherein the MAb that specifically binds to domain 1 of CD6 comprises the sequences SEQ ID NO 1 and 2 or having 98% identity to SEQ ID Nos: 1 and 3.

5. The use according to claim 1, wherein the MAb that specifically binds to domain 1 of CD6 comprises the sequences SEQ ID NO 4 and 5 or having 98% identity to said SEQ ID Nos: 4 and 5.

6. The use according to claim 5, wherein the MAb that specifically binds to domain 1 of CD6 comprises the sequences SEQ ID NO 4 and 5 or having 98% identity to SEQ ID Nos: 4 and 6.

7. The use according to any of the claims 1-6, wherein the conditions that trigger or affect the medical procedures are selecting from the group comprising:
- cardiac surgery, lung transplant surgery, giant brain aneurysm surgery, vascular surgery, extracorporeal membrane oxygenation, mechanical circulatory assistance, haemofiltration, renal dialysis and haemodiafiltration, damage control surgery, trauma, polytrauma, burns, necrotizing peritonitis, syndrome of acute inflammatory response.

8. The use according to any of the claims 1-6, in combination with agents selecting from the group comprising: anesthetics, sedatives, relaxants, antivirals, antibiotics, erythropoietin, agents that block the interaction between IL-6 and IL-6 receptor, and agents that block the interaction between IL-1 and IL-1 receptor.

9. The use according to claim 8 wherein the agents to be combined with the anti-CD6 MAb involve the serial or simultaneous administration.

10. The use according to any of the claims 1-9, wherein the anti-CD6 MAb is a humanized IgG1 MAb.

11. The use according to claim 10, wherein the anti-CD6 MAb is itolizumab.

12. A method of treatment of a subject undergo to extracorporeal circulation (ECC) that comprises the administration by intravenous or subcutaneous route of the MAb comprising the amino acid sequences shown in SEQ ID NO. 1 and 2 or an amino acid sequence having 98% of identity to said sequences comprising the SEQ ID NO. 1 and SEQ ID NO. 3 ranging from 0.3 mg/Kg to 6 mg/Kg of body weight.

13. A method of treatment of a subject undergoing to extracorporeal circulation (ECC) that comprises the administration by intravenous or subcutaneous route of the MAb comprising the amino acid sequences shown in SEQ ID NO. 4 and 5 or an amino acid sequence having 98% of identity to said sequences comprising the SEQ ID NO. 4 and SEQ ID NO. 6 ranging from 0.3 mg/Kg to 6 mg/Kg of body weight.

14. The method according to any of the claims 12-13, wherein the anti-CD6 MAb is administered to the subject at least one time and the time spaced between two consecutives administration is three to seven days.
